# EUROPEAN PATENT APPLICATION

(11) **EP 4 763 277 A1**
(43) Date of publication of application: **24.06.2026**
(21) Application number: 24222158.8
(22) Date of filing: 20.12.2024
(51) Int. Cl.: A61N 5/10, A61B 6/04

(54) **RADIATION THERAPY PATIENT HANDLING SYSTEM**

(71) Applicant: Siemens Healthineers AG, 91301 Forchheim (DE)
(72) Inventor: STOCK, Johannes, 95478 Kemnath (DE); HRUSCHKA, Klaus, 95478 Kemnath (DE); BAUMANN,Berthold, 95478 Kemnath (DE); EIGNER, Daniel, 95478 Kemnath (DE); BUCHENAUER, Andreas, 95478 Kemnath (DE); BARTHEL, Markus, 95478 Kemnath (DE); KNODT, Konstantin, 95478 Kemnath (DE); NEUBER, Wolfgang, 95478 Kemnath (DE)
(74) Representative: Siemens Healthineers Patent Attorneys

(57) **Abstract**

The invention relates to a radiation therapy patient handling system (1), comprising a bedside (11) for a patient, a wagon (8) that is coupled to the bedside (11), and an axis frame module (6) comprising a first beam (12), a second beam (13), a first end cap (15) connecting the first beam (12) and the second beam (13) to each other, and a second end cap (16) also connecting the first beam (12) and the second beam (13) to each other, wherein the first beam (12) and the second beam (13) extend along a longitudinal direction (9) of the axis frame module (6), wherein the wagon (8) is movable within the axis frame module (6) along the longitudinal direction (9), wherein the first beam (12) and the second beam (13) each comprise integrated guidance interfaces (28) extending along the longitudinal direction (9), and wherein the wagon (8) is coupled to the axis frame module (6) by means of the guidance interfaces (28).

## Description

The present invention relates to a radiation therapy patient handling system.

The increasing number of cancer diagnoses every year has increased the demand for modern treatment facilities that are capable of providing an increasing number of patients access to adequate treatment schemes. A commonly used treatment method for cancer therapy is radiation therapy wherein tumor cells are at least locally irradiated by a suitable radiation beam such as, e.g., X-rays, gamma rays, protons, heavy ions, etc.

During radiation therapy, it is essential that a patient to be treated can be positioned with a high position accuracy and a high repeat accuracy. For this purpose, patient handling systems comprising a bedside that can be moved in several degrees of freedom simultaneously can be used. However, for this precise movement, complex structures or assemblies are needed. This makes it difficult to connect assemblies appropriately and robustly and requires a large number of different standardized parts. Complex assembly processes and adjustments are necessary to ensure appropriate axis accuracy - especially with regard to radiotherapy systems - and, above all, repeat accuracy in the manufacture of the patient handling system.

It is thus one object of the present invention to provide an improved radiation therapy patient handling system.

Accordingly, a radiation therapy patient handling system is suggested. The radiation therapy patient handling system comprises a bedside for a patient, a wagon that is coupled to the bedside, and an axis frame module comprising a first beam, a second beam, a first end cap connecting the first beam and the second beam to each other, and a second end cap also connecting the first beam and the second beam to each other, wherein the first beam and the second beam extend along a longitudinal direction of the axis frame module, wherein the wagon is movable within the axis frame module along the longitudinal direction, wherein the first beam and the second beam each comprise integrated guidance interfaces extending along the longitudinal direction, and wherein the wagon is coupled to the axis frame module by means of the guidance interfaces.

The radiation therapy patient handling system is usable for positioning the patient before, during and/or after radiation therapy. For this purpose, the patient rests on the bedside. The bedside can be moved in a plurality of degrees of freedom to position the patient with a high position accuracy as well as with a high repeat accuracy. The bedside preferably is plate shaped. The bedside can be coupled to the wagon via a pitch/roll unit. By means of the pitch/roll unit, the bedside can be pitched and rolled. The wagon enables a linear movement of the bedside along the longitudinal direction. The pitch/roll unit is an optional part or module of the radiation therapy patient handling system. In the case there is no pitch/roll unit, the bedside can be directly attached to the wagon. Apart from that, different modules or assemblies can be used to connect or couple the bedside to the wagon.

The axis frame module can receive any other form of a wagon, for example only a pitch or a module including yaw. The axis frame module can receive in its longitudinal direction any other form of patient support like a couch board, a treatment chair, receivers that are adapted to the body shape of the patient, for example a breast board. The wagon is the interface between the longitudinal direction and the patient support.

A "wagon" in the present case is to be understood as a module or assembly that can perform a transitional or linear movement along the axis frame module. The wagon in particular moves along the longitudinal direction together with the bedside. The wagon is preferably suspended at the axis frame module by means of longitudinal guidances or the like. These longitudinal guidances can guide the wagon along the longitudinal direction. The wagon can be named table wagon. The wagon being movable "within" the axis frame module in the present case means that the axis frame module surrounds or encloses the wagon at least partially. In other words, the axis frame module runs around the wagon. This does not exclude that the wagon can protrude over the axis frame module or out of the axis frame module.

The axis frame module preferably has a rectangular frame-shape. The first beam and the second beam preferably form long sides of the rectangular frame-shape. The first end cap and the second end cap preferably form short sides of the rectangular frame-shape. The first beam and the second beam preferably have an identical cross-section. However, the first beam and the second beam preferably are arranged mirror inverted. The beams are preferably extruded profiles, made of a metal alloy, in particular an Aluminum alloy. The beams can be named profile beams, extrudes profile beams or extruded beams. In particular, the beams are highly integrated extruded profiles. This means, that a plurality of interfaces, cavities or the like that run along the longitudinal direction can be integrated into the beams. The end caps are preferably made of bent sheet metal. The end caps can be formed as integral parts. Alternatively, the end caps can comprise several parts that are connected together. The end caps are preferably bolted to the beams. The beams and the end caps are arranged perpendicular to each other. The first end cap is attached to first end faces of both beams, whereas the second end cap is attached to second end faces of both beams. The first end face of each beam faces away from the second end face. The beam can also be formed as a single integral part. In this case, no end caps are necessary.

The first beam and the second beam are connected to each other only or solely by means of the first end cap and the second end cap. This means that there is preferably no additional connection between the first beam and the second beam apart from the first end cap and the second end cap. There is an additional interface available to connect the first beam and the second beam on an additional level to increase stiffness. The axis frame module forms a stiff and robust frame structure with only a few components in the form of the two beams and the two end caps. More specifically, the axis frame module only comprises four parts. The axis frame module simultaneously creates all necessary interfaces for connected moving axes, cable routing and cover attachments. The axis frame module provides a hygienic design, limits pinch points, and provides the possibility of a simple, noise-reduced and high-performance axis movement. The axis frame module ensures easy accessibility for serviceability and the use of optimized components in form of the beams as rigidity carriers.

The first beam and the second beam "extending or running along the longitudinal direction" means that both beams stretch or extend along the longitudinal direction. In other words, a biggest or longest spatial extension of the first beam and the second beam extends along the longitudinal direction. Preferably, the axis frame module has a coordinate system comprising a first spatial direction, which is identical to the longitudinal direction, a second spatial direction or width direction, and a third spatial direction or height direction. The spatial directions are arranged perpendicular to each other. When seen along the width direction, the first beam and the second beam are arranged spaced apart from each other. However, the first beam and the second beam run parallel to each other. There is a gap provided between the first beam and the second beam. Within this gap, the wagon is placed. The first end cap and the second end cap run throughout the gap for connecting the first beam to the second beam. In the case that there is a single integral beam, the wagon sits within or on the structure of the beam.

The guidance interfaces of the first beam and the second beam being "integrated" into the beams means that the guidance interfaces are part of the beams. The first beam has its own guidance interface, whereas the second beam has its own guidance interface as well. Thus, the first beam and its guidance interface form a single or integral part. The second beam and its guidance interface form a single or integral part as well. Each guidance interface can be formed as a groove that is integrated into the first beam and the second beam. The wagon being "coupled" to the axis frame module by means of the guidance interfaces means that the coupling that is used to connect the wagon to the axis frame module enables a transitional or linear movement of the wagon relatively to the axis frame module. For example, longitudinal guidances can be provided for this purpose. These longitudinal guidances can be connected to the guidance interfaces, wherein the wagon is connected to the longitudinal guidances. However, any other form of coupling that enables a transitional or linear movement of the wagon relative to the axis frame module can be used to couple the wagon to guidance interfaces of the axis frame module.

The use of highly integrated extruded profiles as first beam and second beam, the design of which takes into account the requirements of the intended use, simplifies the intended use and virtually eliminates the possibility of incorrect assembly. The service life of the beams can be simulated and designed according to the requirements by means of upstream calculations regarding stiffness and strength. During the design phase of the beams, all the necessary interfaces can already be taken into account and can therefore all be found on a single component in the form of the beams. These interfaces can be precisely mapped in downstream processes by machining and are therefore a relatively precise fit.

Sufficient technical and clinical cleanliness can already be ensured on the beams. This improves the possibilities for product cleanliness in later field use by the operator. The design as a single part over the entire length of the longitudinal direction avoids external edges, gaps and sealing points. Appropriate contours can also be incorporated to protect operators and users. This eliminates the need for attachment parts, which are essential in conventional designs. The number of components and the assembly time are significantly reduced, making cost-efficient and reliable products possible. Highly load-bearing components with favorable external dimensions are possible. This creates modular interfaces that can be used in a standardized way for adjacent constructions.

The axis frame module is a connected profile structure that forms an interface for all attachments. The axis frame module has a uniform outer surface to improve clinical cleanliness. The beams provide a narrow support structure like cast parts. A repeatable assembly of the axis frame module is possible. Repeatable axis movement is also possible with additional flange-mounted axes. A uniform parameterization is possible. The modular design of the axis frame module is cost-reduced and robust. The simplified assembly due to reduced component scope leads to a cost advantage. Integrated interfaces on a highly integrated component in the assembly are advantageous for e.g. exact movement of the patient along the longitudinal direction. An advanced design is possible, which is a sales argument. A complex geometry can be implemented in each beam. Narrow components make patient handling easier for the operator, which is also a sales argument. Component simulation effort is reduced and closer to reality.

According to an embodiment, a first longitudinal guidance is connected to the guidance interface of the first beam, wherein a second longitudinal guidance is connected to the guidance interface of the second beam, and wherein both longitudinal guidances are connected to the wagon.

The guidance interfaces can receive threaded plates. The longitudinal guidances can be bolted to these threaded plates. In other words, the first longitudinal guidance can be bolted to the first beam and the second longitudinal guidance can be bolted to the second beam. The wagon is attached to the first longitudinal guidance as well as to the second longitudinal guidance.

According to a further embodiment, each longitudinal guidance comprises a guidance rail that is connected to one of the beams and a guidance rail receiver that is connected to the wagon, wherein each guidance rail receiver receives one of the guidance rails, and wherein the guidance rail receivers are guided on the guidance rails along the longitudinal direction.

Preferably, the guidance rails are attached to the guidance interfaces of the beams. More specifically, the guidance rails are bolted to the threaded plates that are received in the guidance interfaces. Preferably, the longitudinal guidances are dovetail guidances. The guidance rail receivers can be bolted to the wagon.

According to a further embodiment, each beam comprises a shoulder surface, wherein each guidance rail lies against one of the shoulder surfaces.

This allows a highly precise positioning of the guidance rails and thus the longitudinal guidances. This helps to create a precise linear movement of the wagon along the longitudinal direction.

According to a further embodiment, the longitudinal guidances are sandwiched between the axis frame module and the wagon such that the axis frame module covers the longitudinal guidances.

The longitudinal guidances being covered by the axis frame module prevents the longitudinal guidances from being soiled by dripping fluids or the likes. This makes cleaning of the longitudinal guidances expandable. Furthermore, there is not removed any lubricant from the longitudinal guidances during cleaning the axis frame module. The longitudinal guidances being "sandwiched" between the axis frame module and the wagon in the present context means that each longitudinal guidance is arranged between the axis frame module and the wagon when seen along the height direction.

According to a further embodiment, the wagon is suspended on the axis frame module by means of the longitudinal guidances.

"Suspended" in this case means that the longitudinal guidances are subjected to tactile forces or tensile forces during operation of the radiation therapy patient handling system. In other words, a weight of the wagon, the bedside, and the patient lasts on the longitudinal guidances in form of tactile forces or tensile forces.

According to a further embodiment, each beam comprises an inner dripping edge protruding over the wagon at least partially, and an outer dripping edge facing away from the inner dripping edge.

In particular, each inner dripping edge protrudes over one of the guidance interfaces and thus the corresponding longitudinal guidance. The inner dripping edge of the first beam and the inner dripping edge of the second beam face the gap being provided between the beams. Thus, the inner dripping edges face each other. The outer dripping edges face away from each other. The dripping edges help the drainage of fluid dripping on the axis frame module. Thus, the fluid drips off from the axis frame module at the dripping edges in a controlled way.

According to a further embodiment, the beams are extruded profiles.

The beams are made of a metal alloy, in particular an Aluminum alloy. This saves weight and provides a high stiffness of the beams. As mentioned before, the beams comprise a plurality of interfaces and a plurality of cavities running along the longitudinal direction.

According to a further embodiment, each beam comprises an outer surface that is coated, in particular lacquered, at least partly.

Thus, additional covers are expendable. This enables a hygienic design. The outer surface forms a visible surface of the axis frame module.

According to a further embodiment, the wagon comprises a wagon interface by means of which the bedside can be coupled to the wagon.

The bedside can be directly connected to the wagon via the wagon interface. However, the bedside can also be connected indirectly to the wagon via the wagon interface. In the latter case, the pitch/roll unit can be connected to the wagon interface, wherein the bedside is connected to the pitch/roll unit. In other words, the pitch/roll unit is sandwiched between the wagon and the bedside. The wagon interface can comprise mechanical connections, for example bolts, electrical connections and/or fluid connections. These connections can also be integrated in the case that there is only provided one beam.

According to a further embodiment, each beam comprises a lower interface and an outer interface, wherein the guidance interfaces, the lower interfaces and/or the outer interfaces are grooves, in particular T-groves, which are integrated into each beam.

The outer interfaces can be used to attach add-on elements, like for example a computer screen, a joystick and/or a table to the axis frame module. The outer interfaces preferably face away from the gap that is provided between the beams. Threaded plates can be inserted into the outer interfaces of the beams. The afore-mentioned add-on elements can be attached to the axis frame module by means of the outer interfaces and the threaded plates being inserted into the outer interfaces. The lower interfaces face downwards. A lower element that closes the axis frame module on its bottom can be attached to the lower interfaces. Preferably, all interfaces are formed into or onto the beams during producing the beams by means of an extrusion process.

According to a further embodiment, the radiation therapy patient handling system further comprises a belt gear for moving the wagon along the longitudinal direction.

The belt gear preferably extends along the longitudinal direction. The belt gear is coupled to the wagon to move the wagon along the longitudinal direction relative to the axis frame module. Using a belt gear enables a lightweight, robust and cost-efficient propulsion of the wagon.

According to a further embodiment, the belt gear comprises a belt that is connected to the wagon, a driven roller that is supported by the first end cap or the second end cap, and a reverse roller that is supported by the first end cap or the second end cap.

The belt is an endless belt that runs around the driven roller and the reverse roller. The belt gear also comprises tensioning rollers to keep tension of the belt constant. Preferably, the belt is clamped between a first clamping element that is firmly attached to the wagon and a second clamping element that is attached to the first clamping element. The driven roller is mounted to the first end cap. The reverse roller is mounted to the second end cap.

According to a further embodiment, the belt gear comprises a motor for driving the driven roller and a brake for decelerating the wagon, wherein the motor and the brake are supported by the first end cap or the second end cap.

The motor preferably is a brushless electric motor. The motor is mounted to the first end cap. The brake can be used for high precision positioning of the patient. Preferably, the brake can be manually opened so that the wagon can be moved along the longitudinal direction manually. This enables to move the patient in case of an emergency, for example.

According to a further embodiment, the radiation therapy patient handling system further comprises flexible cable ducts that are connected to the wagon.

Electric cables can be guided through the cable ducts to the wagon, for example.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

Further embodiments, features and advantages of the present invention will become apparent from the subsequent description and dependent claims, taken in conjunction with the accompanying drawings, in which:
Fig. 1 shows a perspective schematic view of one embodiment of a radiation therapy patient handling system;
Fig. 2 shows a perspective schematic view of one embodiment of an axis frame module for the radiation therapy patient handling system according to Fig. 1;
Fig. 3 shows a front view of one embodiment of a beam for the axis frame module according to Fig. 2;
Fig. 4 shows a cross-sectional view of the radiation therapy patient handling system according to Fig. 1;
Fig. 5 shows a perspective cross-sectional view of the radiation therapy patient handling system according to Fig. 1;
Fig. 6 shows an enlarged perspective cross-sectional view of the radiation therapy patient handling system according to Fig. 1;
Fig. 7 shows another enlarged perspective cross-sectional view of the radiation therapy patient handling system according to Fig. 1;
Fig. 8 shows a schematic cross-sectional view of the radiation therapy patient handling system according to Fig. 1; and
Fig. 9 shows a schematic back view of the radiation therapy patient handling system according to Fig. 1.

In the Figures, like reference numerals designate like or functionally equivalent elements, unless otherwise indicated.

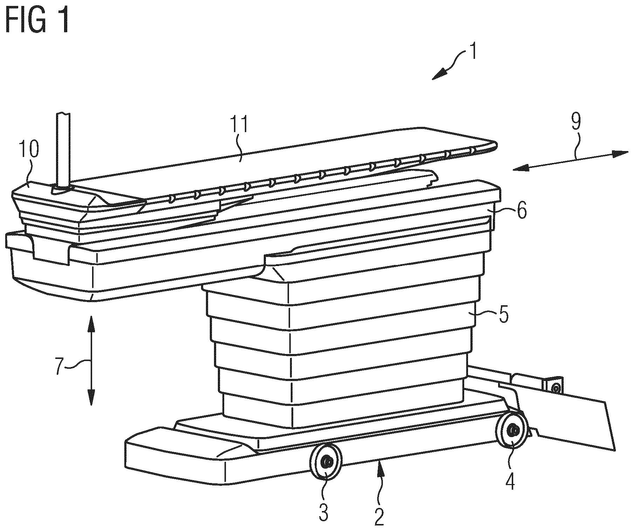

Fig. 1 shows a perspective schematic view of one embodiment of a radiation therapy patient handling system 1.

The radiation therapy patient handling system 1 is used for positioning a patient before and/or during radiation therapy. The radiation therapy patient handling system 1 comprises a foot element 2 with wheels 3, 4 for moving the radiation therapy patient handling system 1 during installation. The foot element 2 carries a telescopable lifting mechanism 5. By means of the lifting mechanism 5, an axis frame module 6 can be lifted and lowered as shown with a double arrow 7 in Fig. 1.

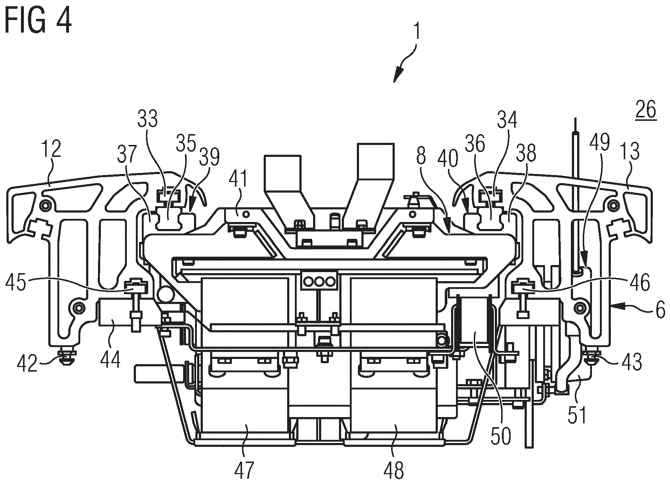

The axis frame module 6 carries a wagon 8 (see Fig. 4) that is movable along a longitudinal direction 9 of the axis frame module 6. The wagon 8 is connected to a pitch/roll unit 10, wherein the pitch/roll unit 10 is connected to a bedside 11 on which the patient rests. By means of the pitch/roll unit 10, the patient together with the bedside 11 can be pitched and/or rolled for positioning the patient.

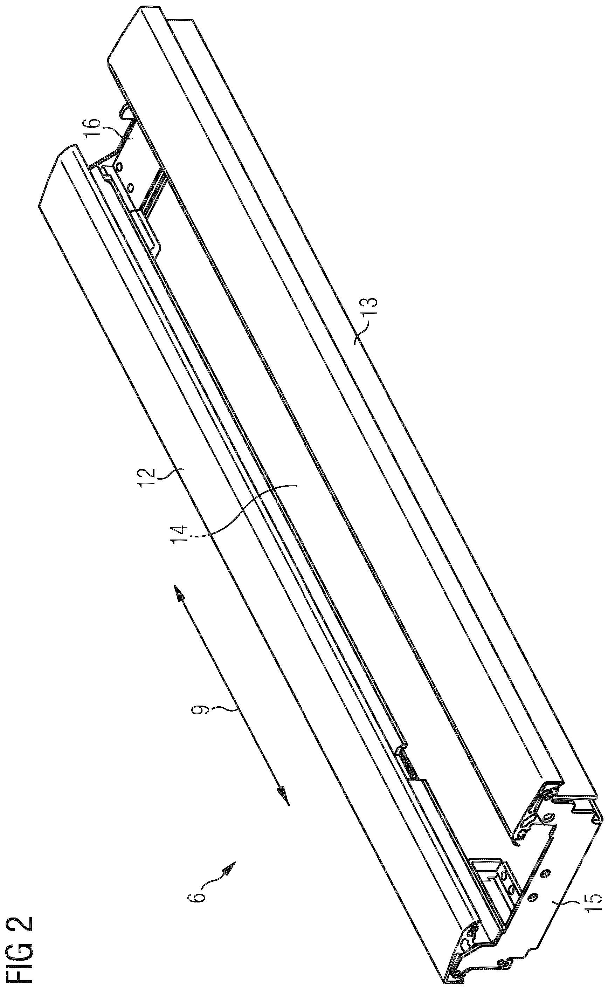

Fig. 2 shows a perspective schematic view of one embodiment of an axis frame module 6 as mentioned before.

The axis frame module 6 comprises a left or first beam 12 and a right or second beam 13. The beams 12, 13 are extrusion molded profiles, preferably made of an aluminium alloy. The beams 12, 13 extend or stretch along the longitudinal direction 9 or vice versa. The beams 12, 13 run parallel to each other and are spaced apart from each other so that a gap 14 is provided between the beams 12, 13. The beams 12, 13 are connected to each other with the aid of a first end cap 15 and a second end cap 16. The first end cap 15 and the second end cap 16 are arranged at two opposing end faces of the beams 12, 13. The end caps 15, 16 are bolted to the beams 12, 13. The beams 12, 13 and the end caps 15, 16 together form a frame-like structure which constitutes the axis frame module 6.

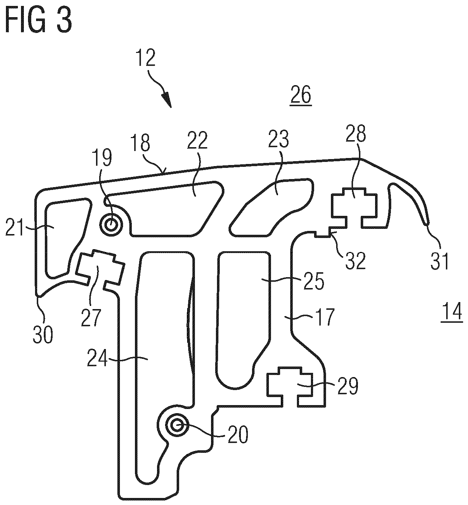

Fig. 3 shows a front view of one embodiment of a first beam 12 as mentioned before.

The beams 12, 13 are identical in geometry but are arranged mirror inverted. In the following, only the first beam 12 will be referred to. All explanations concerning the first beam 12 can be applied to the second beam 13 and vice versa. Fig. 3 shows a view perpendicular to an end face 17 of the first beam 12. The first beam 12 has two end faces 17 facing away from each other. On each end face 17, one of the end caps 15, 16 is mounted.

As mentioned before, the first beam 12 is an extrusion molded profile made by means of a continuous extrusion molding process. The first beam 12 comprises an outer surface 18. The outer surface 18 is coated, in particular lacquered. The first beam 12 comprises inserts 19, 20. Bolts can be screwed into the inserts 19, 20 to attach the first end cap 15 to the first beam 12. Facing away from the inserts 19, 20, additional inserts (not shown) can be provided to attach the second end cap 16 to the first beam 12.

The first beam 12 encloses a plurality of cavities 21, 22, 23, 24, 25 that propagate along the longitudinal direction 9. The cavities 21, 22, 23, 24, 25 are closed toward a surroundings 26 of the first beam 12. There can also be openings provided that open the cavities 21, 22, 23, 24, 25 toward the surroundings 26. The first beam 12 further comprises a plurality of interfaces 27, 28, 29. The interfaces 27, 28, 29 are open toward the surroundings 26. The interfaces 27, 28, 29 can be formed as grooves, in particular T-grooves. In other words, the term "interface" can be exchanged by the term "groove" and vice versa. The interfaces 27, 28, 29 can have a T-shape. Thus, the interfaces 27, 28, 29 can be named T-interfaces. The first beam 12 comprises an outer interface 27, a guidance interface 28 and a lower interface 29.

Furthermore, the first beam 12 comprises an outer dripping edge 30, which faces away from the gap 14, and an inner dripping edge 31 which faces the gap 14. Liquid poured on the outer surface 18 drips off both dripping edges 30, 31 and is prevented from running along the first beam 12 downwards. The first beam 12 also comprises a shoulder surface 32 running along the longitudinal direction 9. The shoulder surface 32 faces the gap 14.

Fig. 4 shows a cross-sectional view of the radiation therapy patient handling system 1.

In Fig. 4, the foot element 2, the lifting mechanism 5, the pitch/roll unit 10, and the bedside 11 are not shown. The guidance interfaces 28 of the beams 12, 13 receive threaded plates 33, 34. The guidance interface 28 of the first beam 12 receives a first threaded plate 33, whereas the guidance interface 28 of the second beam 13 receives a second threaded plate 34. Guidance rails 35, 36 are bolted to the threaded plates 33, 34. A first guidance rail 35 is bolted to the first threaded plate 33, whereas a second guidance rail 36 is bolted to the second threaded plate 34. The guidance rails 35, 36 lie against the shoulder surfaces 32 of the beams 12, 13 for exact positioning of the guidance rails 35, 36. The first guidance rail 35 is received in a first guidance rail receiver 37 which is attached to the wagon 8. The second guidance rail 36 is received in a second guidance rail receiver 38 which is attached to the wagon 8.

The first guidance rail 35 and the first guidance rail receiver 37 form a first longitudinal guidance 39 for the wagon 8. The second guidance rail 36 and the second guidance rail receiver 38 form a second longitudinal guidance 40 for the wagon 8. By means of the longitudinal guidances 39, 40, the wagon 8 is guided along the longitudinal direction 9 within the axis frame module 6.

The wagon 8 carries a wagon interface 41. The wagon interface 41 can be used to connect the wagon 8 to different units. For example, the pitch/roll unit 10 can be connected to the wagon 8 by means of the wagon interface 41.

Connecting elements 42, 43 are attached to the beams 12, 13. The connecting elements 42, 43 can have a mushroom-shape. By means of the connecting elements 42, 43, a cover (not shown) can be mounted to the axis frame module 6. Threaded plates (not shown) can be inserted into the outer interfaces 27 of the beams 12, 13. Add-on elements, like for example a computer screen, a joystick and/or a table, can be attached to the axis frame module 6 by means of the outer interfaces 27 and the threaded plates being inserted into the outer interfaces 27.

The radiation therapy patient handling system 1 further includes a lower element 44. The lower element 44 is attached to the beams 12, 13 by means of threaded plates 45, 46 being received in the lower interfaces 29 of the beams 12, 13. The lower element 44 is bolted to the threaded plates 45, 46. The wagon 8 can move along the longitudinal direction 9 relatively toward the lower element 44. In other words, the lower element 44 stands still, whereas the wagon 8 moves.

The lower element 44 carries flexible cable ducts 47, 48. Furthermore, the lower element 44 at least partly receives a belt gear 49 for moving the wagon 8 along the longitudinal direction 9. The belt gear 49 comprises a belt 50 and a brake 51. The cable ducts 47, 48 are also attached to the wagon 8. If the wagon 8 moves along the axis frame module 6, the cable ducts 47, 48 are deformed or deflected. The cable ducts 47, 48 receive cables, pipes or the like.

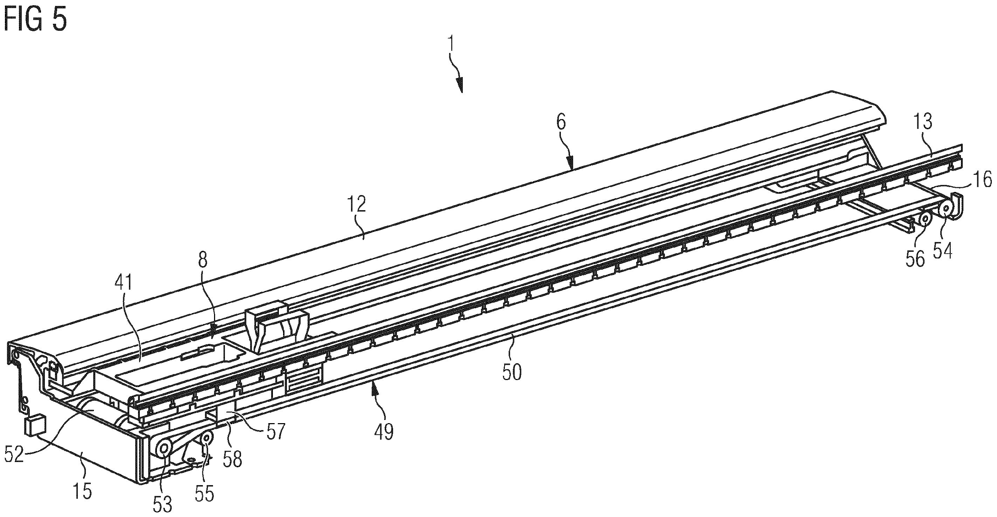

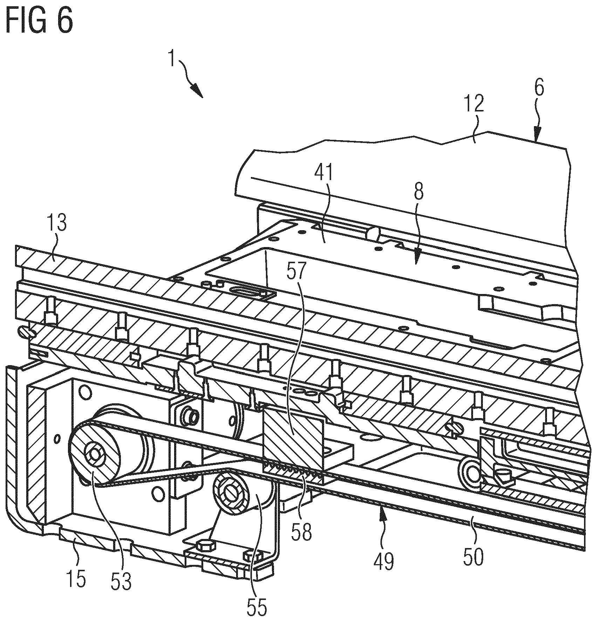

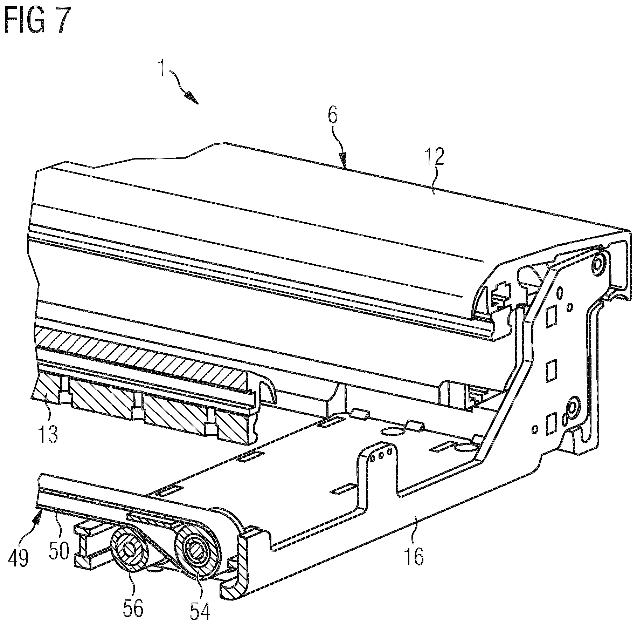

Fig. 5 shows a perspective cross-sectional view of the radiation therapy patient handling system 1. Fig. 6 shows an enlarged perspective cross-sectional view of the radiation therapy patient handling system 1. Fig. 7 shows another enlarged perspective cross-sectional view of the radiation therapy patient handling system 1. In the following, Figs. 5 to 7 will be referred to at the same time.

Besides the belt 50 and the brake 51, the belt gear 49 comprises a motor 52 for driving the belt 50. More precisely, the motor 52 drives a driven roller 53. The motor 52 and the driven roller 53 can be mounted at the first end cap 15. The driven roller 53 drives the belt 50. The belt 50 runs over a reverse roller 54 which is mounted at the second end cap 16. From the reverse roller 54, the belt 50 runs back to the driven roller 53. The belt 50 is an endless belt.

A first tensioning roller 55 is associated with the driven roller 53. A second tensioning roller 56 is associated with the reverse roller 54. The tensioning rollers 55, 56 keep the belt 50 in position and under tension. The belt 50 is clamped between a first clamping element 57 that is firmly attached to the wagon 8 and a second clamping element 58 that is bolted to the first clamping element 57.

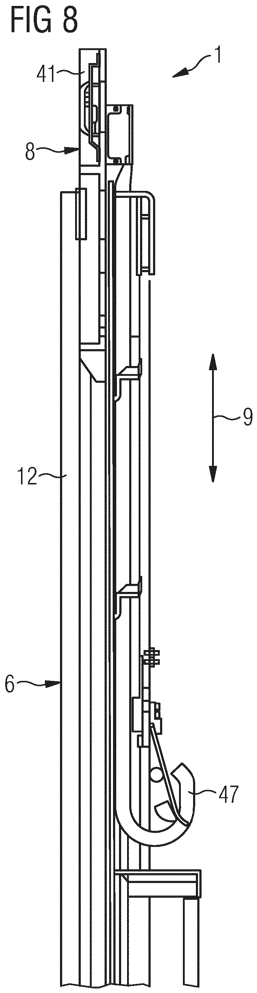

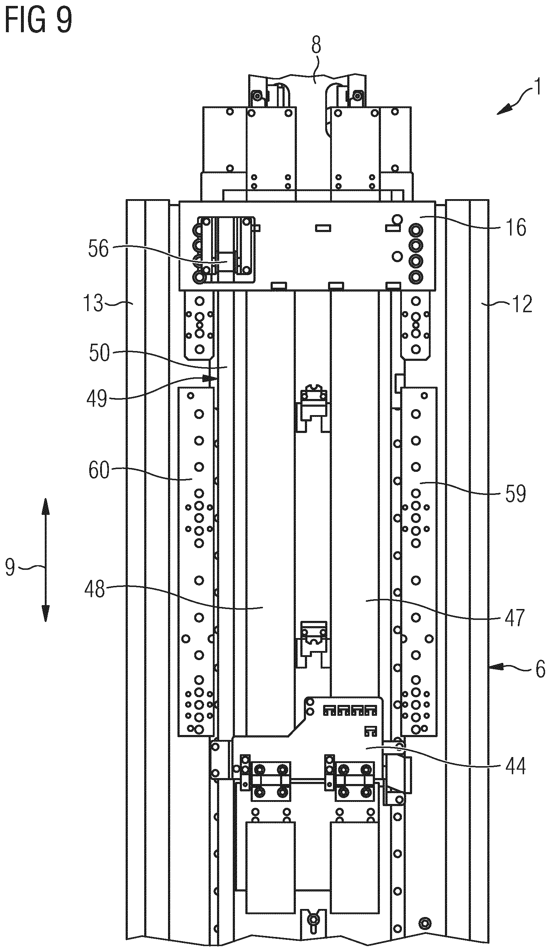

Fig. 8 shows a schematic cross-sectional view of the radiation therapy patient handling system 1. Fig. 9 shows a schematic back view of the radiation therapy patient handling system 1. In the following, Figs. 8 and 9 will be referred to at the same time.

The axis frame module 6 comprises interfaces 59, 60 that can be used to attach arbitrary modules to the axis frame module 6. For example, the lifting mechanism 5 can be attached to the axis frame module 6 by means of the interfaces 59, 60. The interfaces 59, 60 can be threaded plates. A first interface 59 is associated with the first beam 12. A second interface 60 is associated with the second beam 13. The interfaces 59, 60 can be attached to the beams 12, 13 by means of threaded plates that are inserted in the lower interfaces 29 and bolts being screwed into these threaded plates.

Although the present invention has been described in accordance with preferred embodiments, it is obvious for the person skilled in the art that modifications are possible in all embodiments.

### List of Reference

- 1: radiation therapy patient handling system
- 2: foot element
- 3: wheel
- 4: wheel
- 5: lifting mechanism
- 6: axis frame module
- 7: double arrow
- 8: wagon
- 9: longitudinal direction
- 10: pitch/roll unit
- 11: bedside
- 12: beam
- 13: beam
- 14: gap
- 15: end cap
- 16: end cap
- 17: end face
- 18: outer surface
- 19: insert
- 20: insert
- 21: cavity
- 22: cavity
- 23: cavity
- 24: cavity
- 25: cavity
- 26: surroundings
- 27: outer interface
- 28: guidance interface
- 29: lower interface
- 30: outer dripping edge
- 31: inner dripping edge
- 32: shoulder surface
- 33: threaded plate
- 34: threaded plate
- 35: guidance rail
- 36: guidance rail
- 37: guidance rail receiver
- 38: guidance rail receiver
- 39: longitudinal guidance
- 40: longitudinal guidance
- 41: wagon interface
- 42: connecting element
- 43: connecting element
- 44: lower element
- 45: threaded plate
- 46: threaded plate
- 47: cable duct
- 48: cable duct
- 49: belt gear
- 50: belt
- 51: brake
- 52: motor
- 53: driven roller
- 54: reverse roller
- 55: tensioning roller
- 56: tensioning roller
- 57: clamping element
- 58: clamping element
- 59: interface
- 60: interface

## Claims

1. A radiation therapy patient handling system (1), comprising:
a bedside (11) for a patient,
a wagon (8) that is coupled to the bedside (11), and
an axis frame module (6) comprising a first beam (12), a second beam (13), a first end cap (15) connecting the first beam (12) and the second beam (13) to each other, and a second end cap (16) also connecting the first beam (12) and the second beam (13) to each other,
wherein the first beam (12) and the second beam (13) extend along a longitudinal direction (9) of the axis frame module (6),
wherein the wagon (8) is movable within the axis frame module (6) along the longitudinal direction (9),
wherein the first beam (12) and the second beam (13) each comprise integrated guidance interfaces (28) extending along the longitudinal direction (9), and
wherein the wagon (8) is coupled to the axis frame module (6) by means of the guidance interfaces (28).

2. The radiation therapy patient handling system according to claim 1,
wherein a first longitudinal guidance (39) is connected to the guidance interface (28) of the first beam (12), wherein a second longitudinal guidance (40) is connected to the guidance interface (28) of the second beam (13), and wherein both longitudinal guidances (39, 40) are connected to the wagon (8).

3. The radiation therapy patient handling system according to claim 2,
wherein each longitudinal guidance (39, 40) comprises a guidance rail (35, 36) that is connected to one of the beams (12, 13) and a guidance rail receiver (37, 38) that is connected to the wagon (8), wherein each guidance rail receiver (37, 38) receives one of the guidance rails (35, 36), and wherein the guidance rail receivers (37, 38) are guided on the guidance rails (35, 36) along the longitudinal direction (9).

4. The radiation therapy patient handling system according to claim 3,
wherein each beam (12, 13) comprises a shoulder surface (32), and wherein each guidance rail (35, 36) lies against one of the shoulder surfaces (32).

5. The radiation therapy patient handling system according to one of claims 2 to 4,
wherein the longitudinal guidances (39, 40) are sandwiched between the axis frame module (6) and the wagon (8) such that the axis frame module (6) covers the longitudinal guidances (39, 40).

6. The radiation therapy patient handling system according to one of claims 2 to 5, wherein the wagon (8) is suspended on the axis frame module (6) by means of the longitudinal guidances (39, 40).

7. The radiation therapy patient handling system according to one of claims 1 to 6, wherein each beam (12, 13) comprises an inner dripping edge (31) protruding over the wagon (8) at least partially, and an outer dripping edge (30) facing away from the inner dripping edge (31).

8. The radiation therapy patient handling system according to one of claims 1 to 7, wherein the beams (12, 13) are extruded profiles.

9. The radiation therapy patient handling system according to one of claims 1 to 8, wherein each beam (12, 13) comprises an outer surface (18) that is coated, in particular lacquered, at least partly.

10. The radiation therapy patient handling system according to one of claims 1 to 9, wherein the wagon (8) comprises a wagon interface (41) by means of which the bedside (11) can be coupled to the wagon (8).

11. The radiation therapy patient handling system according to one of claims 1 to 10, wherein each beam (12, 13) comprises a lower interface (29) and an outer interface (27), and wherein the guidance interfaces (28), the lower interfaces (29) and/or the outer interfaces (27) are grooves, in particular T-groves, that are integrated into each beam (12, 13).

12. The radiation therapy patient handling system according to one of claims 1 to 11, further comprising a belt gear (49) for moving the wagon (8) along the longitudinal direction (9).

13. The radiation therapy patient handling system according to claim 12,
wherein the belt gear (49) comprises a belt (50) that is connected to the wagon (8), a driven roller (53) that is supported by the first end cap (15) or the second end cap (16), and a reverse roller (54) that is supported by the first end cap (15) or the second end cap (16).

14. The radiation therapy patient handling system according to claim 13,
wherein the belt gear (49) comprises a motor (52) for driving the driven roller (53) and a brake (51) for decelerating the wagon (8), and wherein the motor (52) and the brake (51) are supported by the first end cap (15) or the second end cap (16).

15. The radiation therapy patient handling system according to one of claims 1 to 14, further comprising flexible cable ducts (47, 48) that are connected to the wagon (8).
